# EUROPÄISCHE PATENTSCHRIFT

(11) **EP 0 919 247 B1**
(45) Veröffentlichungstag und Bekanntmachung des Hinweises auf die Patenterteilung: **07.01.2004**
(21) Anmeldenummer: 98122232.6
(22) Anmeldetag: 23.11.1998
(51) Int. Cl.: A61L 2/18

(54) **Verfahren zur chemothermischen Wäschedesinfektion**
Thermochemical disinfection of laundry
Désinfection thermochimique de lessive

(30) Priorität: 28.11.1997 DE 19752801
(43) Veröffentlichungstag der Anmeldung: 02.06.1999
(73) Patentinhaber: Degussa AG, 40474 Düsseldorf (DE)
(72) Erfinder: Reinold, Andreas, 63584 Gründau (DE); Thiele, Georg Dr., 63452 Hanau (DE)

(56) Entgegenhaltungen:
- EP-A- 0 626 371
- WO-A-91/03590
- DE-A- 19 642 127
- US-A- 4 051 059

## Beschreibung

Die Erfindung betrifft ein Verfahren zur chemothermischen Wäschedesinfektion unter Verwendung eines Peressigsäure (PES) und Wasserstoffperoxid (H₂O₂) enthaltenden Desinfektions- und Bleichmittels, insbesondere einer Gleichgewichts-Peressigsäure.

Peressigsäure ist als wirksames Desinfektionsmittel im medizinischen und Lebensmittelsektor, ferner bei der Wasserbehandlung und in gewerblichen Wäschereien bekannt.

Der Einsatz von Peressigsäure erfolgt bei der chemothermischen Wäschedesinfektion in gewerblichen Wäschereien, üblicherweise in Form einer Gleichgewichts-Peressigsäure (G-PES) unterschiedlicher Zusammensetzung - siehe Reiniger + Wäscher XXXVI, Heft 9/83, 21-27. Eine G-PES enthält außer Peressigsäure (PES), Essigsäure, Wasserstoffperoxid (H₂O₂), Wasser und üblichen Aktivsauerstoffstabilisatoren im allgemeinen auch den bei der Herstellung eingesetzten sauren Katalysator, meistens einen Mineralsäurekatalysator. Peressigsäure und Wasserstoffperoxid enthaltende wäßrige Lösungen, insbesondere sogenannte Gleichgewichtsperessigsäuren, wirken sowohl desinfizierend als auch bleichend und zwar bei niedrigen Waschtemperaturen, üblicherweise bei 30 bis 70 °C. Die Einsatzmenge an Peressigsäure in üblichen gewerblichen Waschverfahren liegt im allgemeinen im Bereich von etwa 30 mg bis etwa 300 mg Peressigsäure pro 1 Waschflotte.

In dem zuvor genannten Dokument werden G-PES-Lösungen mit einem Peressigsäuregehalt von 10 Gew.-%, 15 Gew.-% und 4 Gew.-% als Bleich- und Desinfektionsmittel für Waschzwecke genannt, die Konzentration an Wasserstoffperoxid läßt sich aber dem Dokument nicht entnehmen. In der Praxis wurde zunächst hauptsächlich eine G-PES mit 4 bis 5 Gew.-% PES und 20 bis 26 Gew.-% H₂O₂ eingesetzt. Neuerdings zeigt sich wegen ihres besseren Wirkstoff-/Preisverhältnisses ein Trend zu G-PES-Lösungen mit etwa 8 bis 10 Gew.-% PES und einem H₂O₂-Gehalt im Bereich von 18 bis 23 Gew.-%. Nachteilig an den letztgenannten G-PES-Lösungen mit 8 bis 10 Gew.-% PES ist ihr gegenüber den 4 bis 5 Gew.-% PES enthaltenden Lösungen geringerer Bleicheffekt. Einer Erhöhung des H₂O₂-Gehalts in der 8 bis 10 gew.-%igen G-PES standen sicherheitstechnische Bedenken entgegen, da mit einer die Handhabung erschwerenden restriktiveren Transporteinstufung gerechnet werden mußte.

Es ist bekannt, daß sich eine G-PES durch Umsetzung von Essigsäure mit wäßrigem Wasserstoffperoxid in Gegenwart eines sauren Katalysators, wie Schwefelsäure oder der Essigsäure selbst, herstellen läßt, wobei sich die Zusammensetzung aus der Gleichgewichtslage ergibt. Es ist auch bekannt, daß das in einer G-PES vorliegende Gewichtsverhältnis von Peressigsäure zu Wasserstoffperoxid auf die in Betracht gezogene Anwendung von maßgeblicher Bedeutung sein kann. Beispielhaft wird auf die WO 94/16110 verwiesen, wonach bei der Desinfektion wäßriger Zuckerlösungen in einer Prozeßstufe eine Lösung mit einem Molverhältnis von H₂O₂ zu PES von 12:1 bis 120:1 und in einer anderen Prozeßstufe eine Lösung mit einem

Molverhältnis von H₂O₂ zu PES von weniger als 10:1, insbesondere weniger als 5:1 eingesetzt wird.

Die WO 91 03590 A beschreibt ein Verfahren gemäß dem Oberbegriff von Anspruch 1. Bei diesem Verfahren liegt die bevorzugte Peroxysäurekonzentration im Bereich von 2-5 Gew.-% und der Wasserstoffperoxidgehalt im Bereich von 0,1-3 Gew.-%.

Die Aufgabe der Erfindung bestand darin, das Verfahren zur chemothermischen Wäschedesinfektion unter Verwendung eines PES und H₂O₂ enthaltenden Desinfektions- und Bleichmittels bezüglich des Bleicheffekts weiter zu verbessern, ohne ein zusätzliches, separat zu dosierendes Bleichmittel einsetzen zu müssen.

Gelöst wird die Aufgabe durch ein Verfahren zur chemothermischen Wäschedesinfektion, umfassend ein- oder mehrstufiges Waschen eines Waschguts aus textilen

Materialien bei einer Waschtemperatur von mindestens 30 °C, wobei ein Peressigsäure und Wasserstoffperoxid enthaltendes Desinfektions- und Bleichmittel in wirksamer Menge, insbesondere einer Menge von mindestens 3 mg Peressigsäure pro 1 Waschflotte zudosiert wird, das dadurch gekennzeichnet ist, daß man als Desinfektions- und Bleichmittel eine Gleichgewichts-Peressigsäure mit einem Gehalt von 8 bis 12 Gew.-% Peressigsäure und 25 bis 40 Gew.-% Wasserstoffperoxid und einem Gewichtsverhältnis von Peressigsäure zu Wasserstoffperoxid im Bereich von 0,2 bis 0,32 verwendet.

Es handelt sich bei dem zu verwendenden Desinfektions- und Bleichmittel um eine Gleichgewichtsperessigsäure.

Der H₂O₂-Gehalt des Desinfektions- und Bleichmittels im Bereich von 30 bis 45 Gew.-%. Bevorzugt liegt das Gewichtsverhältnis PES zu H₂O₂ im Bereich von 0,25 bis 0,3. Besonders bevorzugt enthält das Desinfektions- und Bleichmittel einen PES-Gehalt im Bereich von 9 bis 11 Gew.-% und einen H₂O₂-Gehalt im Bereich von etwa 35 bis 40 Gew.-%. Ein besonders bevorzugtes Desinfektions- und Bleichmittel enthält 9 bis 11 Gew.-% PES und 35 bis 40 Gew.-% H₂O₂, läßt sich noch unter Einsatz einer etwa 50 gew.-%igem Wasserstoffperoxidlösung herstellen, was aus ökonomischer Sicht vorteilhafter ist als die Verwendung einer höher konzentrierten H₂O₂-Lösung.

Das Verfahren zur Wäschedesinfektion umfaßt allgemein übliche Schritte; es kann diskontinuierlich oder kontinuierlich, jeweils ein oder mehrstufig, durchgeführt werden. Bezüglich der gängigen Waschverfahren in der gewerblichen Wäscherei, welche eine Bleiche und eine Desinfektion umfassen, wird auf Tenside Surfactants Detergents 24 (1987) Nr. 6, 341-349 verwiesen. Übliche Waschprozesse umfassen eine oder mehrere zeitlich und/oder räumlich von nachfolgenden Stufen getrennte Vorwaschstufen, eine oder mehrere Klarwaschstufen und eine oder mehrere Spülstufen. Während übliche Waschmittel oder Netzmittel zu mindestens einer der Vorwaschstufen und/oder mindestens einer Klarwaschstufe zugefügt wird, ist es zweckmäßig, das Desinfektions- und Bleichmittel erst einer Klarwaschstufe zuzudosieren, weil bei einer zu frühzeitigen Zugabe sonst Eiweißverschmutzungen auf der Faser fixiert und Flecken gebildet werden können.

Die Desinfektions- und Bleichmitteldosierung richtet sich nach dem Verschmutzungs- und Verkeimungsgrad. Üblicherweise liegt nach Zugabe des Mittels die PES-Konzentration in der Waschflotte bei mindestens 3 mg/l bis 300 mg/l. Vorzugsweise liegt die Dosierung, berechnet als Peressigsäure (PES) und bezogen auf 1 kg trockenen Waschguts, im Bereich von 20 bis 1000 mg PES, besonders bevorzugt im Bereich von 100 bis 500 mg PES pro kg Waschgut. Die auf das Waschgut bezogene Dosierung gilt vorzugsweise bei einem Flottenverhältnis im Bereich von etwa 1 zu 4 bis 1 zu 7. Bei einem höheren Flottenverhältnis, etwa 1 zu 10 bis 1 zu 20, wie es insbesondere bei Feinwaschverfahren zur Anwendung gelangt, kann es zweckmäßig sein, eine Dosis im oberen Bereich zu verwenden oder die Dosis weiter zu erhöhen, so daß nach Zugabe des Desinfektions- und Bleichmittels die PES-Konzentration in der Waschflotte auch im Bereich von oberhalb 50 mg bis etwa 300 mg PES pro l Waschflotte liegen kann. Bei Bedarf, insbesondere dann, wenn das Spülwasser nicht als Vorspülwasser zum Einsatz gelangt, kann auch dem Spülwasser selbst das Desinfektions- und Bleichmittel zugegeben werden, wobei dann die Dosierung eher im unteren Bereich liegt.

Das erfindungsgemäße Verfahren wird bei einer Waschtemperatur von mindestens 30 °C bis Kochtemperatur durchgeführt, vorzugsweise bei 30 bis 80 °C, insbesondere 30 bis 70 °C. Die Temperaturen richten sich auf jene Stufen, in welchen das zugegebene Desinfektions- und Bleichmittel seine Wirkung entfalten soll.

Das zu verwendende Desinfektions- und Bleichmittel mit einem PES-Gehalt von 8 bis 12 Gew.-% und einem H₂O₂-Gehalt von 25 Gew.-% bis 40 Gew.-%, vorzugsweise 30 Gew.-% bis 40 Gew.-%, mit einem PES/H₂O₂-Gewichtsverhältnis im Bereich von 0,2 bis 0,32 fällt, wie sicherheitstechnische Untersuchungen ergaben, überraschenderweise unter die gleiche Transporteinstufung (5.2 Zi. 9b, UN 3109 - untersucht für eine bevorzugte Lösung mit 10 Gew.-% PES und 37,5 Gew.-% H₂O₂) wie vorbekannte Mittel mit niedrigerem H₂O₂-Gehalt für den genannten Zweck.

Das Desinfektionsmittel D gemäß WO 94/16110 enthält H₂O₂ und PES im Molverhältnis von kleiner 10 zu 1, insbesondere kleiner 5 zu 1, entsprechend einem PES/H₂O₂-Gewichtsverhältnis von größer 0,22 , insbesondere größer 0,44; der H₂O₂-Gehalt wird mit maximal 30 Gew.-% angegeben.

Die erfindungsgemäß zu verwendenden Mittel lassen sich in gleicher Weise herstellen wie vorbekannte PES und H₂O₂ enthaltende Lösungen, insbesondere Gleichgewichtsperessigsäuren: Wäßriges H₂O₂, insbesondere solches mit einem Gehalt von etwa 40 bis 70 Gew.-%, wird mit Essigsäure oder Acetanhydrid in Gegenwart eines sauren Katalysators, etwa Schwefelsäure, bis zur Gleichgewichtseinstellung umgesetzt.

Es wurde gefunden, daß die Einsatzmenge an erfindungsgemäß zu verwendenden Desinfektions- und Bleichmittel gegenüber marktüblichen Mitteln mit ähnlichem PES-Gehalt aber geringerem H₂O₂-Gehalt ohne nennenswerte Minderung der Desinfektionswirkung wesentlich reduziert werden kann. Die geforderte Desinfektionswirkung wird mit einer geringeren Einsatzmenge an PES pro kg Waschgut bzw. Waschflotte erzielt; gleichzeitig wird die Bleichwirkung erhöht. Wie dargelegt, kann das Mittel in gleicher Weise transportiert und gehandhabt werden wie vorbekannte Peressigsäure und H₂O₂ enthaltende wäßrige Lösungen.

Die Erfindung wird anhand des nachfolgenden Beispiels und Vergleichsbeispiels näher erläutert.

### Beispiel 1: Herstellung des Desinfektionsmittels

418 g 50 gew.-%iges wäßriges Wasserstoffperoxid, 70 g Essigsäure, 5,2 g Wasser, 4,9 g Schwefelsäure, 0,05 g Dipicolinsäure und 2,0 g 10 gew.-%ige wäßrige Hydroxyethandiphosphonsäure-Lösung wurden gemischt und bei Raumtemperatur 2 Tage stehengelassen. Die resultierende G-PES enthielt 10,4 Gew.-% PES und 37,2 Gew.-% H₂O₂ (die Bestimmung von PES und H₂O₂ erfolgte in bekannter Weise durch zweistufige Titration mittels Cer(IV)sulfat und Natriumthiosulfat); der gesamte Aktivsauerstoffgehalt betrug 19,7 Gew.-%.

### Beispiel 2 und Vergleichsbeispiel: Waschversuch

Durchgeführt wurde ein Waschversuch unter Verwendung des Desinfektionsmittels von Beispiel 1 (= Beispiel 2) und des im Markt erhältlichen Produktes Ozonit® Super der Firma Henkel (= Vergleichsbeispiel).

Durchgeführt wurden die Versuche in einer Waschstraße einer kontinuierlichen Waschmaschine vom Typ Senking P19 mit 20 Waschstufen (einschließlich Vorwäsche und Spülung) mit je 2 Minuten Verweildauer. Das Desinfektions-/Bleichmittel wurde bei der maximalen Waschtemperatur von 60 °C der Stufe 9 zudosiert. Der PES- und H₂O₂-Gehalt konnte zwecks Optimierung der Einsatzmenge an Desinfektions-/Bleichmittel mittels Reflectoquant®-Teststäbchen ermittelt werden.

Der Vorwäsche (6 Stufen inclusive einer Netzungsstufe) wurden ein Netzmittel (0,5 g / kg Waschgut) und ein übliches aktivsauerstofffreies Waschmittel (5,8 g / kg Waschgut) zugegeben, Waschmittel zusätzlich noch unmittelbar vor der Desinfektionsmittelzugabe. Das Flottenverhältnis betrug 5:1, das Waschgut war normal verschmutzte Krankenhauswäsche. Der pH-Wert zum Zeitpunkt der Desinfektionsmittelzugabe lag im Bereich von 9 bis 10.

Die Zusammensetzung der Desinfektions-/Bleichmittel, die Einsatzmenge sowie physikalische Prüfergebnisse und mikrobiologische Prüfergebnisse folgen aus der Tabelle.

Es zeigte sich, daß das erfindungsgemäße Desinfektionsmittel trotz 40 % geringerer Einsatzmenge zu hervorragenden Bleich- und Desinfektionsergebnissen führt.

So wird die gegenüber dem Stand der Technik erfindungsgemäß erhältliche Steigerung des Grundweißwertes (Y-Wert; Weißwert unter Eliminierung der von optischen Aufhellern bewirkten Weißgradsteigerung) als bedeutsam bewertet (Anforderung: Y > 85). Auch die geringerer Farbtonabweichungszahl (FAZ) ist von technischer Bedeutung. Während im mikrobiologischen Abklatsch der Feuchtwäsche unter Einsatz des erfindungsgemäßen Mittels Keimzahlen unterhalb der zulässigen Grenze gefunden wurden, war die trockene Wäsche keimfrei; bei Verwendung des vorbekannten Mittels (Vergleichsbeispiel) war der KBE-Wert für die Feuchtwäsche 0, dagegen waren zwei Proben der Trockenwäsche nicht keimfrei. Daraus folgt, daß die Desinfektionswirkung im Beispiel 2 und im Vergleichsbeispiel etwa gleich war.

**Tabelle**

| Versuchsergebnisse bei Krankenhauswäsche ***) | Vergleichsbeispiel | Beispiel 2 |
|---|---|---|
| **Desinfektionsmittel (DM)** | Ozonit® Super | Beispiel 1 |
| Gehalt PES (%) | 9,00 | 10,4 |
| Gehalt H₂O₂ (%) | 23,6 | 37,2 |
| Gehalt ES (%) | 8,7 | 6,00 |
| Gehalt AO (%) | 13,00 | 19,7 |
| Einsatzmenge (ml DM/kg Trockenwäsche) | 5,00 | 3,00 |
| **Physikalische Prüfergebnisse** | | |
| Reißkraftverlust (%) | 7,5 | 6,3 |
| Schädigungsfaktor (%) | 0,4 | 0,5 |
| Glühasche (%) | 0,2 | 0,1 |
| Weißgrad (WG-Wert) | 210 | 213 |
| Farbtonabweichungszahl (FAZ) | G 0,80 | G 0,50 |
| Grundweißwert (Y-Wert) | 92 | 93 |
| **Mikrobiologische Abklatsche** | | |
| Feuchtwäsche (KBE/dm²) *) | 0 | 8 und 20 |
| trockene Wäsche (KBE/dm²) **) | n10 | n10 |
| | (0/0/0/4/0/0/0/0/4/0) | (0/0/0/0/0/0/0/0/0/0) |
| **Keimträger** | | |
| Keimträgerläppchen (Wachstum Staphylococcus) | negativ | negativ |
| Keimträgerläppchen (Wachstum Enterococcus) | negativ | negativ |
| KBE = koloniebildende Einheiten (Keimzahl) | | |

| | | |
|---|---|---|
| *) Anforderung: max. 30 KBE/dm² | | |
| **) Anforderung: max. 20 KBE/dm² | | |
| ***) Untersuchungen gemäß Güte- und Prüfbestimmungen RAL-RG 992/1 "Sachgemäße Wäschepflege" und Mikrobiologische Kontrolle gemäß RAL-RG 992/2 "Krankenhauswäsche" | | |

## Patentansprüche

1. Verfahren zur chemothermischen Wäschedesinfektion, umfassend ein- oder mehrstufiges Waschen eines Waschguts aus textilen Materialien bei einer Waschtemperatur von mindestens 30 °C, wobei ein Peressigsäure und Wasserstoffperoxid enthaltendes Desinfektions- und Bleichmittel in wirksamer Menge, insbesondere einer Menge von mindestens 3 mg Peressigsäure pro l Waschflotte zudosiert wird,
**dadurch gekennzeichnet,**
**daß** man als Desinfektions- und Bleichmittel eine Gleichgewichts-Peressigsäure mit einem Gehalt von 8 bis 12 Gew.-% Peressigsäure und 25 bis 40 Gew.-% Wasserstoffperoxid und einem Gewichtsverhältnis von Peressigsäure zu Wasserstoffperoxid im Bereich von 0,2 bis 0,32 verwendet.

2. Verfahren nach Anspruch 1,
**dadurch gekennzeichnet,**
**daß** das Gewichtsverhältnis Peressigsäure zu Wasserstoffperoxid der zuzudosierenden Gleichgewichts-Peressigsäure im Bereich von 0,25 bis 0,3 liegt.

3. Verfahren nach Anspruch 1 oder 2,
**dadurch gekennzeichnet,**
**daß** die zuzudosierende Gleichgewichts-Peressigsäure 9 bis 11 Gew.-% Peressigsäure und 35 bis 40 Gew.-% Wasserstoffperoxid enthält.

4. Verfahren nach einem der Ansprüche 1 bis 3,
**dadurch gekennzeichnet,**
**daß** man das Desinfektions- und Bleichmittel in einer Menge entsprechend 20 bis 1000 mg Peressigsäure, insbesondere 100 bis 500 mg Peressigsäure pro kg trockenen Waschguts zudosiert.

5. Verfahren nach einem der Ansprüche 1 bis 4,
**dadurch gekennzeichnet,**
**daß** man das Waschen in einer eine oder mehrere Vorwaschstufen, eine oder mehrere Klarwaschstufen und eine oder mehrere Spülstufen umfassenden kontinuierlichen Waschstraße durchführt, wobei man zu einer Vorwaschstufe und/oder zu einer Klarwaschstufe ein bleichmittelfreies Waschmittel zugibt und zu einer der nachfolgenden Klarwaschstufen das Desinfektions- und Bleichmittel bei einer Temperatur im Bereich von 30 bis 80 °C zudosiert.

## Claims

1. Process for chemothermal disinfection of laundry, comprising single- or multi-stage washing of laundry consisting of textile materials at a washing temperature of at least 30°C, an effective quantity of a disinfectant and bleaching agent containing peracetic acid and hydrogen peroxide being dosed in, in particular a quantity of at least 3 mg peracetic acid per 1 washing liquor,
**characterised in that**
an equilibrium peracetic acid containing 8 to 12 wt.% peracetic acid and 25 to 40 wt.% hydrogen peroxide and having a weight ratio of peracetic acid to hydrogen peroxide in the range of 0.2 to 0.32 is used as the disinfectant and bleaching agent.

2. Process according to claim 1,
**characterised in that**
the weight ratio of peracetic acid to hydrogen peroxide in the equilibrium peracetic acid to be dosed in is in the range of 0.25 to 0.3.

3. Process according to claim 1 or 2,
**characterised in that**
the equilibrium peracetic acid to be dosed in contains 9 to 11 wt.% peracetic acid and 35 to 40 wt.% hydrogen peroxide.

4. Process according to one of claims 1 to 3,
**characterised in that**
the quantity of disinfectant and bleaching agent dosed in is equivalent to a quantity of 20 to 1000 mg peracetic acid, in particular 100 to 500 mg peracetic acid per kg dry laundry.

5. Process according to one of claims 1 to 4,
**characterised in that**
washing is carried out in a continuous washing system comprising one or more pre-wash stages, one or more main wash stages and one or more rinsing stages, a bleach-free detergent being added to a pre-wash and/or a main wash stage and the disinfectant and bleaching agent being dosed into one of the subsequent main wash stages at a temperature in the range of 30 to 80°C.

## Revendications

1. Procédé de désinfection thermochimique de lessive comprenant un lavage à une ou plusieurs étapes d'un article à laver en matériau textile à une température de lavage d'au moins 30°C, à laquelle on ajoute un agent de désinfection et de blanchiment contenant de l'acide peracétique et du peroxyde d'hydrogène en quantité efficace, en particulier une quantité d'au moins 3 mg d'acide peracétique par bain de lavage,
**caractérisé en ce que**
comme agent de désinfection et de blanchiment on utilise un acide peracétique en équilibre avec une teneur de 8 à 12 % en poids d'acide peracétique et 25 à 40 % en poids de peroxyde d'hydrogène et un rapport pondéral d'acide peracétique à peroxyde d'hydrogène dans l'intervalle de 0,2 à 0,32.

2. Procédé selon la revendication 1,
**caractérisé en ce que**
le rapport pondéral acide peracétique à peroxyde d'hydrogène de l'acide peracétique en équilibre à ajouter se situe dans l'intervalle de 0,25 à 0,3.

3. Procédé selon la revendication 1 ou 2,
**caractérisé en ce que**
l'acide peracétique en équilibre à ajouter contient de 9 à 11 % en poids d'acide peracétique et de 35 à 40 % en poids de peroxyde d'hydrogène.

4. Procédé selon l'une des revendications 1 à 3,
**caractérisé en ce que**
l'agent de désinfection et de blanchiment est ajouté en une quantité correspondant à 20 à 1 000 mg d'acide peracétique, en particulier 100 à 500 mg d'acide peracétique par kg d'articles à laver sec.

5. Procédé selon l'une des revendications 1 à 4,
**caractérisé en ce que**
le lavage s'effectue dans une station de lavage en continu comprenant une ou plusieurs étapes de prélavage, une ou plusieurs étapes de lavage à l'eau claire et une ou plusieurs étapes de rinçage, en ajoutant à une étape de prélavage et/ou à une étape de lavage à l'eau claire un agent de lavage exempt d'agent de blanchiment, et en dosant à une des étapes suivantes de lavage à l'eau claire, l'agent de désinfection et de blanchiment à une température dans l'intervalle de 30 à 80°C.
